(19) Europäisches Patentamt
European Patent Office
Office européen des brevets

(11) Veröffentlichungsnummer: **0 034 723**
**B1**

(12) # EUROPÄISCHE PATENTSCHRIFT

(45) Veröffentlichungstag der Patentschrift: **18.05.83**

(51) Int. Cl.[3]: **C 07 C 41/26,** C 07 C 43/295

(21) Anmeldenummer: **81100665.9**

(22) Anmeldetag: **30.01.81**

(54) **Verfahren zur Herstellung von 4-Phenoxyphenolen.**

(30) Priorität: **08.02.80 DE 3004610**

(43) Veröffentlichungstag der Anmeldung:
**02.09.81 Patentblatt 81/35**

(45) Bekanntmachung des Hinweises auf die Patenterteilung:
**18.05.83 Patentblatt 83/20**

(84) Benannte Vertragsstaaten:
**AT BE CH DE FR GB IT LI NL**

(56) Entgegenhaltungen:
**CH-A-428 758**
**DE-A-2 648 644**
**GB-A-1 402 446**

**CHEMICAL ABSTRACTS, Band 89, Nr. 21, November 1978, Zusammenfassung Nr. 179698w COLUMBUS, Ohio (US)**

**Houben-Weyl Methoden der organischen Chemie 4. Auflage 1976, Band 6/1c, Seite 256**

(73) Patentinhaber: **HOECHST AKTIENGESELLSCHAFT, Postfach 80 03 20, D-6230 Frankfurt/Main 80 (DE)**

(72) Erfinder: **Volkwein, Gert, Dr., Im Schulzehnten 7, D-6233 Kelkheim(Taunus) (DE)**
Erfinder: **Schönowsky, Hubert, Dr., Siegweg 1, D-6074 Rödermark (DE)**
Erfinder: **Baessler, Konrad, Dr., Drosselweg 1, D-6230 Frankfurt am Main 80 (DE)**

**EP 0 034 723 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von 4-Phenoxyphenolen

Gegenstand der Erfindung ist ein Verfahren zur Herstellung von 4-Phenoxyphenolen der Formel

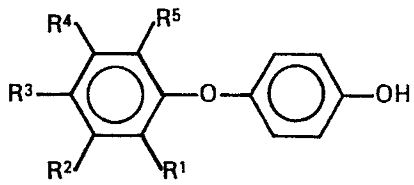

I

in der $R^1$ bis $R^5$ unabhängig voneinander Wasserstoff- oder Halogenatome oder niedere Alkylgruppen bedeuten, durch Diazotierung eines Amins der Formel

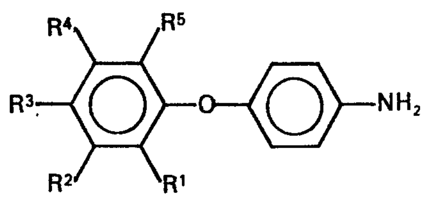

II.

in der $R^1$ bis $R^5$ die genannten Bedeutungen haben, in einem wässrigen mineralsauren Medium und Verkochen in wässriger Schwefelsäure in Gegenwart eines mit Wasser nicht mischbaren, das entstehende Phenol lösenden Lösemittels, dadurch gekennzeichnet, dass die Diazotierung in Salzsäure und die Verkochung bei einer Temperatur von 110 bis 150°C erfolgt.

Aus der DE-PS 21 36 828 ist es bereits bekannt, Phenoxyphenole der obengenannten Art durch Diazotieren des entsprechenden Amins in Gegenwart von Salzsäure und Verkochen des Diazoniumsalzes herzustellen. Hierbei wird das Amin zunächst in Eisessig gelöst, dann durch Zugabe von Salzsäure das Hydrochlorid ausgefällt und mit wässriger Natriumnitritlösung diazotiert. Anschliessend wird mit Borfluorwasserstoffsäure das Diazoniumfluoborat abgetrennt, welches in Essigsäureanhydrid verkocht wird. Aus der so erhaltenen Acetoxyverbindung wird das Phenol durch Verseifen mit methanolischer Natronlauge und Ansäuern mit Salzsäure freigesetzt.

Die Abtrennung des Diazoniumsalzes als Fluoborat erfolgt offensichtlich zur Entfernung der Chloridionen, da diese bekanntermassen in einer Nebenreaktion der Phenolverkochung zu einer Kernchlorierung führen (Houben-Weyl, 4. Auflage 1976, Band 6/1c, Seite 249), und zwar um so mehr, je höher die Halogenkonzentration ist [J. Amer. chem. Soc. 91 (1969) 2, 430].

Ein verbessertes Verfahren zur Herstellung solcher 4-Phenoxyphenole ist in der DE-AS 26 48 444 beschrieben, wobei Diazotierung und Verkochung in 60- bis 75%iger Schwefelsäure erfolgen, bevorzugt in Gegenwart katalytischer Mengen von Alkalichloriden.

Aus der SU-PS 391 128 ist es bekannt, 4,4'-Diaminodiphenylether mit einem Gemisch aus Schwefelsäure und Salzsäure (3,7 g $H_2SO_4$ und 0,4 g HCl pro g Ether) zu diazotieren. Die Verkochung erfolgt in 50%iger Schwefelsäure unter Rückfluss (130°C). Bei dieser im Verhältnis zum aus der DE-PS 26 48 644 bekannten Verfahren wesentlich erhöhten Konzentration an Chloridionen wird hier nur eine Ausbeute von 51,5% an 4,4'-Dihydroxydiphenylether erhalten. Es bestand also ein erhebliches Vorurteil gegen die Diazotierung solcher Amine in Gegenwart von Salzsäure.

Überraschenderweise wurde jedoch gefunden, dass bei dem erfindungsgemässen Verfahren die schon sehr guten Ausbeuten gemäss DE-PS 26 48 644 noch verbessert werden können, wobei zusätzlich die Raumausbeute erhöht wird. Erstaunlicherweise werden auch trotz der hohen Chloridkonzentration im Verkochungsgemisch die Produkte in hoher Reinheit gebildet; es tritt also kein nennenswerter Diazonium-Halogen-Austausch ein.

Im folgenden werden bevorzugte Ausgestaltungen der Erfindung näher erläutert.

In bevorzugten Ausgangsmaterialien stehen $R^1$ bis $R^5$ für Wasserstoff-, Chlor- und/oder Bromatome. Besonders bevorzugt sind Ausgangsmaterialien, in denen $R^1$ für Wasserstoff oder Chlor, $R^2$, $R^4$ und $R^5$ für Wasserstoff und $R^3$ für Wasserstoff oder Chlor stehen.

Im folgenden beziehen sich Prozentangaben auf das Gewicht, wenn nichts anderes ausgesagt ist.

Zur Diazotierung wird das Amin in 2,1 bis 5 Mol 10- bis 36%ige Salzsäure eingetragen, um das Hydrochlorid herzustellen, das sich in der Hitze löst und beim Abkühlen unter Rühren eine gut rührbare Suspension ergibt. Zu dieser Suspension wird dann festes oder in Wasser gelöstes Alkalimetallnitrit, insbesondere Natriumnitrit, bei 0 bis 30°C zugegeben.

Die Verkochung erfolgt durch Zugabe der vom Nitritüberschuss befreiten Diazoniumchloridlösung zu einem siedenden Gemisch aus einer in der Regel mindestens 60%igen Schwefelsäure und einem mit Wasser nicht mischbaren Lösemittel für das Produkt. Als solche Lösemittel kommen insbesondere aromatische Kohlenwasserstoffe wie Toluol und Xylol in Betracht. Die Reaktionstemperatur der unter Rückfluss kochenden Reaktionsmischung wird annähernd konstant gehalten, indem entweder Wasser ausgekreist oder konzentrierte Schwefelsäure zugegeben wird. Bei Temperaturen bis etwa 150°C werden Ausbeuten von mindestens 93% der Theorie erreicht, wobei die Rückflusstemperatur durch eine entsprechende Schwefelsäurekonzentration eingestellt werden kann.

Bevorzugt erfolgt die Verkochung bei 115 bis 125°C.

Bei tieferen Verkochungstemperaturen nimmt die Bildung von Nebenprodukten stark zu, so

dass die Ausbeute entsprechend abfällt und eine aufwendige Reinigung erforderlich wird. Bei einer Verkochungstemperatur von 100 bis 105°C entstehen schon über 5% Halogenierungsprodukte.

Wegen der im allgemeinen leichteren Diazotierbarkeit und sehr viel höheren Löslichkeit der Diazoniumchloride werden gegenüber der Diazotierung in Schwefelsäure höhere Ausbeuten und insbesondere höhere Raumausbeuten erhalten. Erfindungsgemäss wird so bis zur Hälfte des nach dem Verfahren gemäss DE-AS 2648644 nicht umgesetzten Amin-Anteils in das Phenol überführt, wobei, bezogen auf das Reaktionsvolumen, die doppelte Aminmenge umgesetzt wird.

Das erfindungsgemässe Verfahren wird in den folgenden Beispielen näher erläutert. Auch hier beziehen sich Prozentangaben auf das Gewicht, wenn nichts anderes angegeben ist.

Beispiel 1

In 400 ml Wasser und 300 g Salzsäure, 30%ig, werden 220 g 4-Chlor-4′-aminodiphenylether eingetragen. Durch Erwärmen auf etwa 100°C wird eine klare Lösung hergestellt, aus der beim Rühren unter Kühlen auf 10°C eine gut rührbare Suspension des Hydrochlorids entsteht, das mit 180 g 40%iger wässriger $NaNO_2$-Lösung diazotiert wird. Ein eventueller Nitritüberschuss wird mit Amidosulfonsäure zerstört.

Zur Verkochung werden 380 ml Wasser, 710 g $H_2SO_4$, konz., und 860 g Xylol vorgelegt und auf 120°C erwärmt. Dann wird die Diazoniumsalzlösung im Lauf von etwa 4 Stunden eingetragen. Die Rückflusstemperatur von mindestens 120°C bleibt dadurch erhalten, dass an einem Wasserabscheider etwa 850 g Wasser ausgekreist werden. Nach 1 Stunde Nachrühren ist die Gasentwicklung beendet. Man lässt unter Rühren abkühlen, trennt die Phasen und extrahiert die Xylollösung mit Natronlauge. Die Natriumsalzlösung des Phenols wird angesäuert und der 4-Chlor-4′-hydroxydiphenylether geschmolzen abgetrennt, mit Wasser gewaschen und getrocknet.

Ausbeute: 206 g 4-Chlor-4′-hydroxydiphenylether (93,3% d.TH.)
EP: 83,0°C
Reingehalt (RG): 99% ± 2% (GC mit innerem Standard)

Unter den gleichen Reaktionsbedingungen wurde auch 4-Aminodiphenylether umgesetzt.

Ausbeute: 93,0% d.Th.
EP: 81,7°C
RG: 96% ± 2% (GC mit innerem Standard)

Beispiel 2

In 600 ml Wasser und 300 g Salzsäure, 30%ig, werden bei 80°C 254 g geschmolzener 2,4-Dichlor-4′-aminodiphenylether eingetragen. Durch Erwärmen auf 95°C erhält man eine klare Lösung, aus der beim Rühren unter Kühlen auf 15°C eine gut rührbare Suspension des Hydrochlorids entsteht. Dieses wird mit 180 g 40%iger wässriger $NaNO_2$-Lösung diazotiert. Eventueller Nitritüberschuss wird mit Amidosulfonsäure zerstört.

Zur Verkochung werden 190 ml Wasser, 480 g $H_2SO_4$, konz., und 1200 ml Xylol vorgelegt und auf 115°C erwärmt. Dann wird die Diazoniumsalzlösung im Lauf von etwa 3 Stunden zugetropft, wobei gleichzeitig 1455 g $H_2SO_4$, konz., so zugegeben werden, dass mindestens 115°C Rückflusstemperatur eingehalten werden können. Nach 1 Stunde Nachrühren bei 115°C ist die Gasentwicklung beendet. Es wird unter Rühren abgekühlt, die Phasen werden getrennt und die Xylollösung wird mit Natronlauge extrahiert. Die Natriumsalzlösung des Phenols wird angesäuert, der 2,4-Dichlor-4′-hydroxydiphenylether wird geschmolzen abgetrennt, mit Wasser gewaschen und getrocknet.

Ausbeute: 243 g 2,4-Dichlor-4′-hydroxydiphenylether (95,3% d.Th.)
EP.: 88,7°C
RG.: 99% ± 2% (GC mit innerem Standard)

**Patentansprüche**

1. Verfahren zur Herstellung von 4-Phenoxyphenolen der Formel I

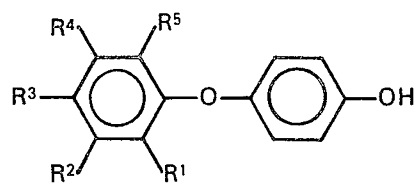

I

in der $R^1$ bis $R^5$ unabhängig voneinander Wasserstoff- oder Halogenatome oder niedere Alkylgruppen bedeuten, durch Diazotierung eines Amins der Formel II

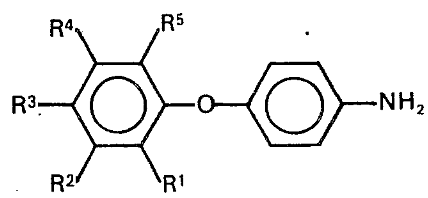

II

in der $R^1$ bis $R^5$ die genannten Bedeutungen haben, in wässriger Mineralsäure und Verkochen in wässiger Schwefelsäure in Gegenwart eines mit Wasser nicht mischbaren, das entstehende Phenol lösenden Lösemittels, dadurch gekennzeichnet, dass die Diazotierung in Salzsäure und die Verkochung bei einer Temperatur von 110 bis 150°C erfolgt.

2. Verfahren nach Anspruch 1, dadurch gekennzeichnet, dass man Amine der Formel I einsetzt, in der $R^1$ und $R^3$ unabhängig voneinander Wasserstoff oder Chlor bedeuten und die übrigen Reste für Wasserstoff stehen.

3. Verfahren nach Anspruch 1 und 2, dadurch

gekennzeichnet, dass die Verkochung bei 115 bis 125°C vorgenommen wird.

4. Verfahren nach Anspruch 1 bis 3, dadurch gekennzeichnet, dass die Diazotierung in 10- bis 36%iger Salzsäure erfolgt.

## Claims

1. A process for the preparation of 4-phenoxyphenols of the formula I

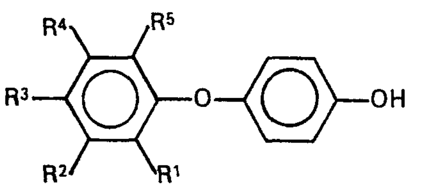

I

in which $R^1$ through $R^5$ independently are hydrogen, halogen or lower alkyl, by diazotization of an amine of the formula II

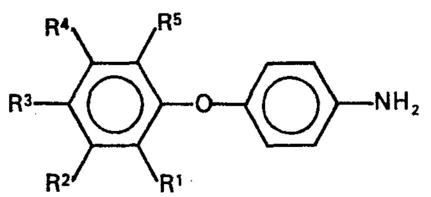

II

in which $R^1$ through $R^5$ are as defined above, in an aqueous mineral acid medium and boiling in aqueous sulfuric acid in the presence of a water-immiscible solvent capable of dissolving the phenol formed, which comprises carrying out the diazotization in hydrochloric acid and the boiling at a temperature of from 110 to 150°C.

2. The process as claimed in claim 1, which comprises using amines of the formula I, in which $R^1$ and $R^3$, independently from each other, are hydrogen or chlorine, and the other radicals are hydrogen.

3. The process as claimed in claims 1 and 2, which comprises carrying out the boiling at 115 to 125°C.

4. The process as claimed in claims 1 to 3, which comprises carrying out the diazotization in 10 to 36% hydrochloric acid.

## Revendications

1. Procédé de préparation de phénoxy-4 phénols répondant à la formule I:

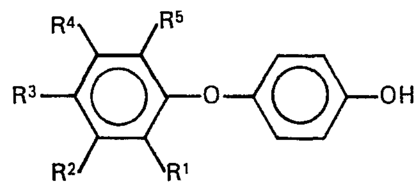

I

dans laquelle $R^1$ à $R^5$ représentent chacun, indépendamment les uns des autres, un atome d'hydrogène, un atome d'halogène ou un radical alkyle inférieur, par diazotation d'une amine répondant à la formule II:

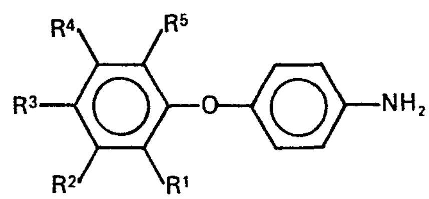

II

dans laquelle $R^1$ à $R^5$ ont les significations précédemment données, dans un acide minéral aqueux, et transformation par ébullition dans de l'acide sulfurique aqueux en présence d'un solvant non miscible à l'eau et capable de dissoudre le phénol en voie de formation, procédé caractérisé en ce qu'on effectue la diazotation dans de l'acide chlorhydrique et la transformation par ébullition à une température de 110 à 150°C.

2. Procédé selon la revendication 1 caractérisé en ce qu'on utilise des amines de formule I dans lesquelles $R^1$ et $R^3$ représentent chacun, indépendamment l'un de l'autre, l'hydrogène ou le chlore, les autres symboles représentant chacun l'hydrogène.

3. Procédé selon l'une des revendications 1 et 2 caractérisé en ce qu'on effectue la transformation par ébullition à une température de 115 à 125°C.

4. Procédé selon l'une quelconque des revendications 1 à 3 caractérisé en ce qu'on effectue la diazotation dans de l'acide chlorhydrique d'une concentration comprise entre 10 et 36%.